# EUROPEAN PATENT APPLICATION

(11) **EP 2 937 098 A1**
(43) Date of publication of application: **28.10.2015**
(21) Application number: 14382150.2
(22) Date of filing: 24.04.2014
(51) Int. Cl.: A61K 38/18, A61K 35/36, A61P 17/14

(54) **A pharmaceutical composition comprising a suspension of total cells obtained from hair follicle and plasma derived growth factors for promoting hair follicle regeneration**

(71) Applicant: Clinica Dermatologica Ercilla, 48009 Bilbao, Vizcaya (ES)
(72) Inventor: Gorrochategui Barrueta, Alberto, 48009 Bilbao, Vizcaya (ES)
(74) Representative: Hoffmann Eitle

(57) **Abstract**

The invention relates to pharmaceutical compositions and methods for treating hair loss and regenerating hair follicles. Specifically, the invention relates to pharmaceutical compositions comprising a suspension of hair progenitor cells obtained from a complete hair follicle and plasma derived growth factors for treating hair loss or regenerating hair follicles.

## Description

### FIELD OF THE INVENTION

The invention relates to pharmaceutical compositions and methods for treating hair loss and regenerating hair follicles. Specifically, the invention relates to pharmaceutical compositions comprising a suspension of hair progenitor cells obtained from a complete hair follicle and plasma derived growth factors for treating hair loss or regenerating hair follicles.

### BACKGROUND OF THE INVENTION

Follicular neogenesis is defined as the generation of new hair follicles (HF) after birth. Humans are born with a full complement of HF, which can change in size and growth characteristics as in early baldness or can ultimately degenerate and disappear as in late stages of baldness or in permanent scarring (cicatricial) alopecias. Therefore, the generation of new HF is desirable in the treatment of common baldness as well as less common hair loss conditions, such as discoid lupus erythematosis, congenital hypotrichosis, lichen planopilaris and other scarring alopecias.

### SUMMARY OF THE INVENTION

In a first aspect of the present invention, the invention provides methods of treating hair loss, treating, inhibiting, or suppressing a degenerative skin disorder, and treating scarring and non-scarring alopecia, such as androgenetic alopecia (AGA), in a subject, comprising the administration to the subject of a composition comprising:
- a suspension of cells, wherein said cell suspension comprises hair progenitor cells (HPCs), obtained from a tissue sample comprising a complete hair follicle from the said subject, and
- plasma derived growth factors, preferably obtained from said subject.

Thus, in one embodiment of the first aspect of the invention, the present invention provides a method of treating hair loss in a subject comprising the steps of (a) obtaining a composition comprising a suspension of cells from a complete hair follicle and plasma derived growth factors, preferably from the said subject, and (b) administering said composition to the subject. In a preferred embodiment, the administering step is subepidermal, intralesional, dermal or epidermal.

In one embodiment of the first aspect of the invention, the hair loss is due to androgenetic alopecia (AGA). In one embodiment of the first aspect of the invention, the AGA is male pattern baldness. In another embodiment, the AGA is female pattern baldness. In one embodiment of the first aspect of the invention, the hair loss is the result of a skin injury. In one embodiment of the first aspect of the invention, the hair loss is in the scalp or eyebrow of said subject. In one embodiment of the first aspect of the invention, the hair loss is in scarred skin tissue of said subject. In one embodiment of the first aspect of the invention, the scarring alopecia is hair implant scarring alopecia. In one embodiment of the first aspect of the invention, the scarring alopecia is acquired by radiation. In one embodiment of the first aspect of the invention, the scarring alopecia is frontal fibrosing scarring alopecia. In one embodiment of the first aspect of the invention, the hair loss is the result of a non-scarring alopecia. In one embodiment of the first aspect of the invention, the non-scarring alopecia is autoimmune non-scarring (Areata) alopecia. In one embodiment of the first aspect of the invention, the non-scarring alopecia is non-scarring follicular miniaturization alopecia. In one embodiment of the first aspect of the invention, the non-scarring alopecia is Effluviums.

In another embodiment of the first aspect of the invention, the present invention provides a method for generating a hair follicle in the skin of a subject with hair loss comprising the steps of (a) obtaining a composition comprising a suspension of cells from a complete hair follicle of the subject and plasma derived growth factors, preferably from said subject, and (b) administering said composition to the subject. In a preferred embodiment, the administering step is via subepidermal, intralesional, dermal or epidermal. In another embodiment of the first aspect of the invention, the present invention provides a method for increasing the size of a hair follicle in the skin of a subject with hair loss comprising the steps of (a) obtaining a composition comprising a suspension of cells from a complete hair follicle of the subject and plasma derived growth factors, preferably from said subject, and (b) administering said composition to the subject. In a preferred embodiment, the administering step is via subepidermal, intralesional, dermal or epidermal. In another embodiment, the present invention provides a method for increasing hair follicle formation in the skin of a subject with hair loss comprising the steps of (a) obtaining a composition comprising a suspension of cells from a complete hair follicle of the subject and plasma derived growth factors from, preferably from said subject, and (b) administering said composition to the subject. In a preferred embodiment, the administering step is via subepidermal, intralesional, dermal or epidermal. In another embodiment, the present invention provides a method for treating, inhibiting, or suppressing a degenerative skin disorder comprising the steps of (a) obtaining a composition comprising a suspension of cells from a complete hair follicle of the subject and plasma derived growth factors, preferably from said subject, and (b) administering said composition to the subject. In a preferred embodiment, the administering step is via subepidermal, intralesional, dermal or epidermal.

A second aspect of the invention refers to a method of obtaining a cell suspension comprising HPCs from a human subject which comprises the following steps:
a. Obtaining a tissue sample comprising at least one complete hair follicle from the subject, preferably from the occipital region of the head of the subject;
b. Enzymatically or mechanically digest the cells from the tissue obtained from the sample of step a);
c. If needed, inactivate the enzyme of step b); and
d. Removing the tissue fragments from the cell suspension obtained from steps b) or c), preferably by filtration.

A preferred embodiment of the second aspect of the invention refers to a method of obtaining a cell suspension comprising HPCs from a human subject which comprises the following steps:
a. Obtaining a tissue sample comprising at least one complete hair follicle from the subject, preferably from the occipital region of the head of the subject;
b. Introducing the tissue obtained from step b in a tube containing collagenase;
c. Incubating the tissue with collagenase for a period of time between 15 and 60 minutes while shaking at a temperature of approximately 37 °C;
d. Inactivating the enzyme of step c; and
e. Removing the tissue fragments from the cell suspension obtained from step d), preferably by filtration.

A preferred embodiment of the second aspect of the invention refers to a method of obtaining a cell suspension comprising HPCs from a human subject which comprises the following steps:
a. Obtaining a tissue sample comprising at least one complete hair follicle from the subject, preferably from the occipital region of the head of the subject;
b. Introducing the tissue in a tube containing 0.075 % w/v of collagenase;
c. Incubating the tissue with the collagenase for a period of time between 15 and 60 minutes while shaking, preferably for a period of approximately 30 minutes, at a temperature of approximately 37 °C;
d. Inactivating the enzyme of step c, preferably with human serum;
e. Removing the tissue fragments from the product of step d); and
f. Centrifuging the product of step e) and removing the supernatant.

A third aspect of the invention refers to a method of obtaining a composition comprising a cell suspension comprising HPCs from a human subject and plasma derived growth factors which comprises the following steps:
a. Obtaining a tissue sample comprising at least one complete hair follicle from the subject, preferably from the occipital region of the head of the subject;
b. Introducing the tissue obtained from step a) in a tube containing an enzyme suitable for the enzymatic digestion of the cells;
c. Incubating the tissue with the enzyme;
d. Inactivating the enzyme of step c);
e. Removing the tissue fragments from the product of step d);
f. Re-suspending the cell composition of step e) in a suspension of plasma derived growth factors, preferably obtained from the subject; and
g. Filtrating the cell suspension obtained from step f).

A preferred embodiment of the third aspect of the invention refers to a method of obtaining a composition comprising a cell suspension comprising HPCs from a human subject and plasma derived growth factors, which comprises the following steps:
a. Obtaining a tissue sample comprising at least one complete hair follicle from the subject, preferably from the occipital region of the head of the subject;
b. Introducing the tissue in a tube containing 0.075 % w/v collagenase;
c. Incubating the tissue with the collagenase for a period of time between 15 and 60 minutes while shaking at a temperature of approximately 37 °C, preferably for a period of approximately 30 minutes;
d. Inactivating the enzyme of step c), preferably with human serum;
e. Removing the tissue fragments from the product of step d);
f. Centrifuging the product of step e) and removing the supernatant;
g. Re-suspending the cell composition of step f) in a suspension of plasma derived growth factors, preferably obtained from the subject; and
h. Filtrating the cell suspension obtained of step g).

A fourth aspect of the invention refers to a method of obtaining a composition comprising a cell suspension comprising HPCs from a human subject and plasma derived growth factors which comprises the following steps:
a. Obtaining a tissue sample comprising at least one complete hair follicle from the subject, preferably from the occipital region of the head of the subject;
b. Incubating the tissue in a suspension of plasma derived growth factors, preferably obtained from the subject; for a period of time between 15 minutes and 1 hour at a temperature of approximately 37 °C;
c. Removing the tissue fragments from the product of step b); preferably by filtration.

A preferred embodiment of the fourth aspect of the invention refers to a method of obtaining a composition comprising a cell suspension comprising HPCs from a human subject and plasma derived growth factors, which comprises the following steps:
a. Obtaining a tissue sample comprising at least one complete hair follicle from the subject, preferably from the occipital region of the head of the subject;
b. Optionally, washing the tissue obtained from step a);
c. Incubating the tissue in a suspension of plasma derived growth factors, preferably obtained from the subject; for a period of time between 15 minutes and 1 hour at a temperature of 37 °C;
d. Homogenizing the cell composition of step c) by means of a mechanical or manual homogenizer such as a Potter homogenizer; and
e. Removing the tissue fragments from the product of step d); preferably by filtration.

A preferred embodiment of the fourth aspect of the invention refers to a method of obtaining a composition comprising a cell suspension comprising HPCs from a human subject and plasma derived growth factors, which comprises the following steps:
a. Obtaining a tissue sample comprising at least one complete hair follicle from the subject, preferably from the occipital region of the head of the subject;
b. Optionally, washing the tissue obtained from step a), preferably the washing step is done more than one time;
c. Incubating the tissue in a suspension of plasma derived growth factors, preferably obtained from the subject; for a period of time between 15 minutes and 1 hour at a temperature of approximately 37 °C;
d. Homogenizing the cell composition of step c) by means of a mechanical or manual homogenizer such as a Potter homogenizer; and
e. Removing the tissue fragments from the product of step d); preferably by filtration.

A preferred embodiment of the fourth aspect of the invention refers to a method of obtaining a composition comprising a cell suspension comprising HPCs from a human subject and plasma derived growth factors, which comprises the following steps
a. Obtaining a tissue sample comprising at least one complete hair follicle from the subject, preferably from the occipital region of the head of the subject;
b. Optionally, washing the tissue obtained from step a), preferably this washing step is done more than one time;
c. Incubating the tissue in a suspension of plasma derived growth factors, preferably obtained from the subject; for a period of time of approximately 30 minutes at a temperature of aproximately 37 °C;
d. Homogenizing the cell composition of step c) by means of a mechanical or manual homogenizer such as a Potter homogenizer; and
e. Removing the tissue fragments from the product of step d); preferably by filtration.

A fifth aspect of the invention refers to a cell suspension comprising hair progenitor cells (HPCs) of a human subject obtained or obtainable by means of the method of the second aspect of the invention.

A sixth aspect of the invention refers to a composition comprising a cell suspension comprising HPCs from a human subject and plasma derived growth factors preferably obtained from the said human subject, obtained or obtainable by means of the method of the third aspect of the invention.

A seventh aspect of the invention refers to a composition comprising a cell suspension comprising HPCs from a human subject and plasma derived growth factors preferably obtained from the said human subject, obtained or obtainable by means of the method of the fourth aspect of the invention.

In an eight aspect of the present invention, the invention provides methods of treating hair loss, treating, inhibiting, or suppressing a degenerative skin disorder, and treating scarring and non-scarring alopecia, such as androgenetic alopecia (AGA), in a subject and generating new hair follicles (HF) and increasing the size of existing HF, comprising the administration of the cell suspension or composition of any of aspects fifth, sixth or seventh. Thus, in one preferred embodiment of the eight aspect of the invention, the present invention provides a method of treating hair loss in a subject comprising the steps of (a) obtaining the composition or the cell suspension of any of aspects fifth, sixth or seventh and (b) administering said composition or cell suspension to the subject. In a preferred embodiment, the administering step is via subepidermal, intralesional, dermal or epidermal.

In one embodiment of the eight aspect of the invention, the hair loss is due to androgenetic alopecia (AGA). In one embodiment of the eight aspect of the invention, the AGA is male pattern baldness. In another embodiment, the AGA is female pattern baldness. In one embodiment of the eight aspect of the invention, the hair loss is the result of a skin injury. In one embodiment of the eight aspect of the invention, the hair loss is in the scalp or eyebrow of said subject. In one embodiment of the eight aspect of the invention, the hair loss is in scarred skin tissue of said subject. In one embodiment of the eight aspect of the invention, the scarring alopecia is hair implant scarring alopecia. In one embodiment of the eight aspect of the invention, the scarring alopecia is acquired by radiation. In one embodiment of the eight aspect of the invention, the scarring alopecia is frontal fibrosing scarring alopecia. In one embodiment of the eight aspect of the invention, the hair loss is the result of a non-scarring alopecia. In one embodiment of the eight aspect of the invention, the non-scarring alopecia is autoimmune non-scarring (Areata) alopecia. In one embodiment of the eight aspect of the invention, the non-scarring alopecia is non-scarring follicular miniaturization alopecia. In one embodiment of the eight aspect of the invention, the non-scarring alopecia is Effluviums.

In another embodiment of the eight aspect of the invention, the present invention provides a method for generating a hair follicle in the skin of a subject with hair loss comprising the steps of (a) obtaining the composition or cell suspension of any of aspects fifth, sixth or seventh and (b) administering the same to the subject. In a preferred embodiment, the administering step is via subepidermal, intralesional, dermal or epidermal. In another embodiment of the eight aspect of the invention, the present invention provides a method for increasing the size of a hair follicle in the skin of a subject with hair loss comprising the steps of (a) obtaining the composition or cell suspension of any of aspects fifth, sixth or seventh and (b) administering the same to the subject. In a preferred embodiment, the administering step is via subepidermal, intralesional, dermal or epidermal. In another embodiment, the present invention provides a method for increasing hair follicle formation in the skin of a subject with hair loss comprising the steps of (a) obtaining the composition or cell suspension of any of aspects fifth, sixth or seventh and (b) administering the same to the subject. In a preferred embodiment, the administering step is via subepidermal, intralesional, dermal or epidermal. In another embodiment, the present invention provides a method for treating, inhibiting, or suppressing a degenerative skin disorder comprising the steps of (a) obtaining the composition or cell suspension of any of aspects fifth, sixth or seventh and (b) administering the composition to the subject. In a preferred embodiment, the administering step is via subepidermal, intralesional, dermal or epidermal.

Other features and advantages of the present invention will become apparent from the following detailed description examples and figures. It should be understood, however, that the detailed description and the specific examples while indicating preferred embodiments of the invention are given by way of illustration only, since various changes and modifications within the spirit and scope of the invention will become apparent to those skilled in the art from this detailed description.

### BRIEF DESCRIPTION OF THE DRAWINGS

**Figure 1****.** Follow-up photographs, JHM1978; a) 29.01.2013; b) 25.03.2013 and c) 11.12.2013.
**Figure 2****.** Follow-up photographs, DZP1981; a) 12.07.2013 and b) 18.10.2013
**Figure 3****.** Follow-up photographs, IIG1982; a) 09.10.2012; b) 26.06.2013; c) 19.12.2013 and d) 5 Months follow-up for the second treatment.
**Figure 4****.** Follow-up photographs, JMM1973; a) 18.04.2013 and b) 10.06.2013.
**Figure 5****.** Follow-up photographs, ZBO1969; a) 05.03.2013; b) 20.06.2013 and c) 12.07.2013
**Figure 6****.** Follow-up photographs, ERA1944; a) 14.10.2013 and b) 11.02.2014
**Figure 7****.** Follow-up photographs, SFR1945; a) 05.04.2013 and b) 11.02.2014.
**Figure 8****.** Follow-up photographs, BUB1972; a) 10.04.2013 and b) 22.05.2013.
**Figure 9****.** Follow-up photographs, BLP1969; a) 09.09.2010 and b) 18.02.2014.

### DESCRIPTION OF THE PRESENT INVENTION

### 1. DEFINITIONS

Alopecia is the fundamental sign of most of the diseases of the scalp. It entails complete hair loss (atrichia) or partial hair loss (hypotrichosis). The different types of alopecia are classified into two large groups:
- Scarring alopecia: this alopecia occurs as the result of an irreversible follicular lesion and it also usually entails damage to the skin involved.
- Non-scarring alopecia: this alopecia entails a reversible alteration of the hair follicle that does not involve the surrounding skin, or an intrinsic lesion of the hair shaft.

Particular forms of scarring and non-scarring alopecia are:
- Androgenetic alopecia (AGA): A progressive, diffuse, symmetric loss of scalp hair, believed due to a combination of genetic predisposition and increased response of hair follicles to androgens, in men beginning around age 30 with hair loss from the vertex and frontoparietal regions, and in females beginning later with less severe hair loss in the frontocentral area of the scalp.
- Hair implant scarring alopecia: It is a destruction of hair follicles by inflammatory processes at the level of the dermis due to previous surgical techniques such as artificial hair or hair transplants.
- Scarring alopecia acquired by radiation: This type of alopecia is developed as a result of the use of radiotherapy for the treatment of brain tumours which in turn destroys the hair follicle.
- Frontal fibrosing scarring alopecia: It is characterised by usually symmetrical band of hair loss on the front and sides of the scalp, and loss of eyebrows. The edge may appear moth-eaten, and single hairs may persist in the bald areas.
- Autoimmune non-scarring (Areata) alopecia: It is a recurrent non-scarring type of hair loss that can affect any hair-bearing area and can manifest in many different patterns. Although it is a benign condition and most patients are asymptomatic, it can cause emotional and psychosocial distress.
- Non-scarring follicular miniaturization alopecia: Alopecia which are characterized by a decrease in follicular activity and a gradual miniaturization of hair cycles.
- Effluviums: Acute, sub-acute or chronic hair loss due to internal or external agents that act on the hair follicles, causing dysfunction of the growth cycle of the follicles.

The term "a tissue sample comprising at least one complete hair follicle" means a tissue sample comprising
1. a hair follicle which in turn comprises at least the following elements a bulged follicle (matrix and dermal papilla), a hair shaft, an inner and outer epithelial sheath, a bulge area and the perifollicular sheath; and
2. preferably a sebaceous gland, apocrine gland and arrector pili muscle

Preferably, the term "a tissue sample comprising at least one complete hair follicle" means a tissue sample comprising a pilosebaceous unit.

The term "HPCs" refers to a suspension of precursor cells capable of generating or differentiating into the different cell types that make up the hair follicle. In the cell suspensions of the present invention, these cells will usually be found in combination with other cells such as epidermal cells (keratinocytes), dermal cells (fibroblasts), subcutaneous cells (adipocytes) as well as other cells from the different regions of the complete hair follicle.

The term "punch" refers to a scalpel comprising a circle of different diameters (from 0.5 to 4 mm, preferably 1.5 mm) suitable for removing a pilosebaceous unit or skin tissue by manual or automatic rotation.

The procedure for obtaining tissue by using a punch would be, with a punch an incision is made in the area where a hair follicle is found in order to obtain a small sample of skin tissue. The punch on the skin surface penetrates the tissue to a depth of 0.8-1 cm by applying a rotational movement.

The term "about" or "approximately" in reference to a numeric value means +/- 10 % of that numeric value. The term "about" in reference to a numeric value also includes +/- 5 % of that numeric value.

The terms "comprise" and "comprising" are used in the inclusive, open sense, meaning that additional elements may be included.

The term comprises also encompasses and may be used interchangeably with the terms "consists of" and "consists essentially of".

### 2. DETAILED DESCRIPTION OF THE INVENTION

The present invention refers to compositions, pharmaceutical compositions and methods for treating hair loss and regenerating hair follicles. Specifically, the invention relates to methods of treating hair loss, treating, inhibiting, or suppressing a degenerative skin disorder , and treating scarring and non-scarring alopecia, such as androgenetic alopecia (AGA), in a subject and generating new hair follicles (HF) and increasing the size of existing HF, comprising the administration of a composition comprising a suspension of total cells obtained from a complete hair follicle, wherein these cell suspension comprises HPCs, and plasma derived growth factors preferably obtained from said subject.

In this sense, the authors of the present invention have discovered that a cell suspension comprising HPCs obtained from a complete hair follicle in combination with plasma derived growth factors, when administered to a human subject suffering from any type of hair loss is capable of a) stabilizing the hair loss; b) increasing capillary density, causing a constant renewal of capillary cycles and regenerating new hairs and c) maintaining and renewing the follicular and cellular structure of the treated area.

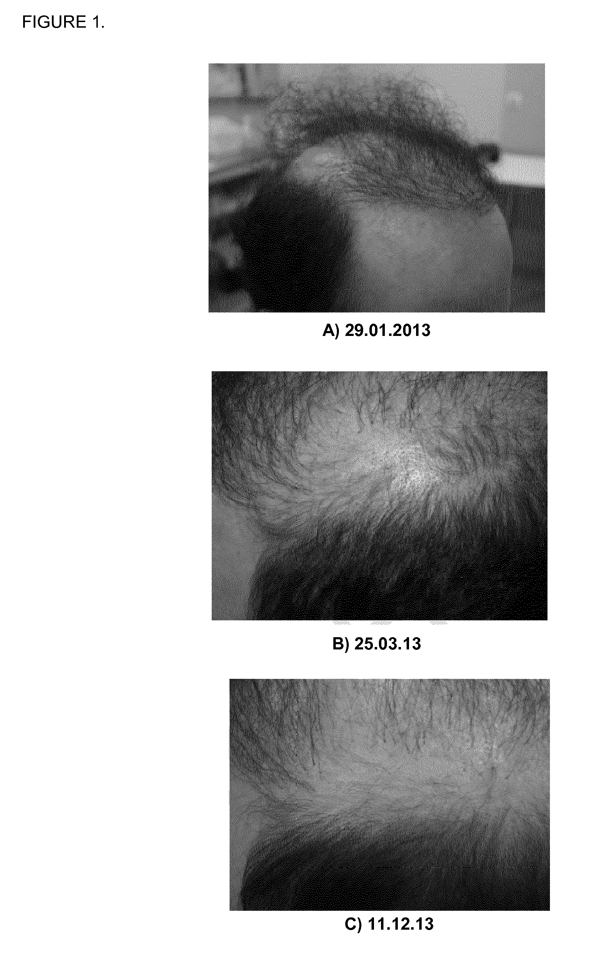

In this regard, the authors of the present invention have used the cell suspension illustrated in step 2 of example 3 to treat a wide variety of alopecia. In this sense, they administered (Infiltration throughout the frontal, parietal and vertex regions) the product of Step 2 of example 3 to a human subject suffering from scarring alopecia acquired by hair implant and as illustrated in tables III and IV and figure 1, the administration of this product caused a progressive regulation and biological renewal in the treated area and surrounding areas, in fact it caused an increased in the number of anagen hairs and an increased in the activity of reserve cells inactive until the treatment started. The fact that this technique is an effective and simple alternative for the treatment of scarring alopecia acquired by hair implant is surprising, since to date there is no effective treatment of this type of alopecia that is not accompanied by adverse effects.

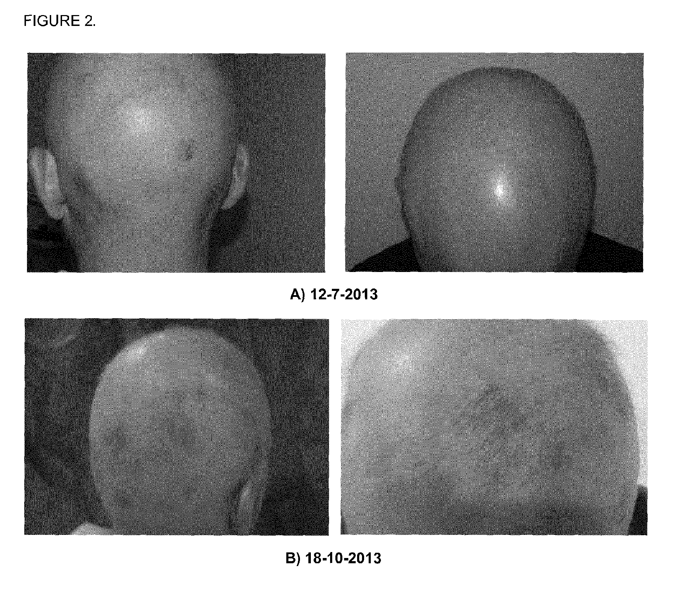

In addition, the authors of the present invention have used the cell composition of the invention in the treatment of non-scarring autoimmune (Areata) alopecia. In this case, the authors of the invention administered subepidermally to a human subject suffering from this disease, the composition product of Step 2 of example 3. As illustrated in table V and also in figure 2, the administration of this product did not produce any adverse effects and after one month from its first administration new hair progressively appeared. In addition, after two months of the first administration increasingly stronger hair was observed in the different regions and surrounding areas were the product was originally applied. For these reasons, this technique is an effective and simple alternative for the treatment of also this type of alopecia for which the current alternatives have shown a great number of contraindications as well as relatively mild effects.

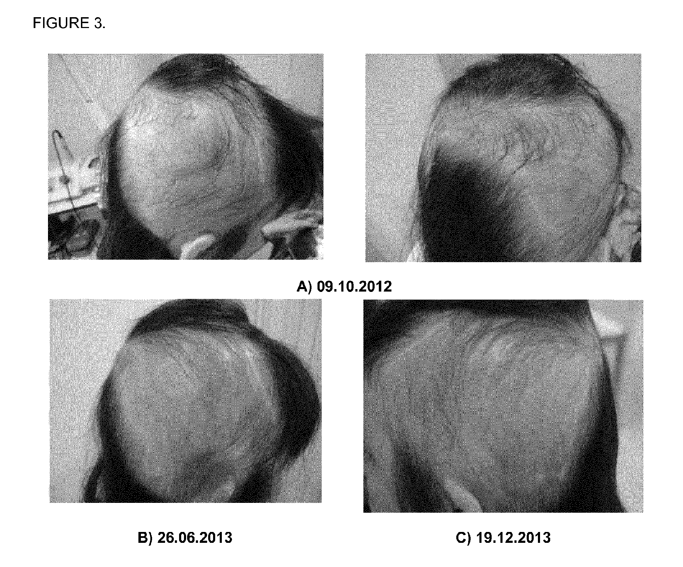

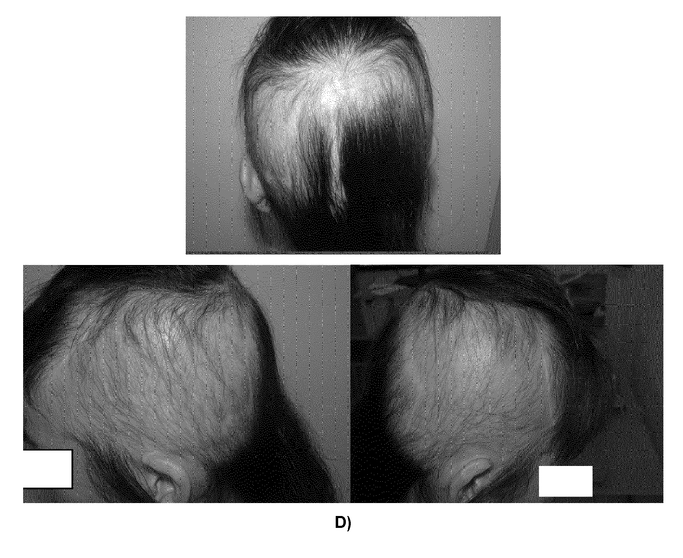

Furthermore, the authors of the present invention have used the cell composition of the invention in the treatment of scarring alopecia acquired by radiation. In this case, the authors of the invention administered subepidermally (infiltration throughout the entire bald area (extensive and diffuse area all over the head)) to a human subject suffering from this disease, the composition product of Step 2 of example 3. As illustrated in tables VI and VII below and also in figure 3, the human subject did not show any adverse effects and surprisingly after two months from the first administration of the composition product of the invention, the cells of the hair follicles inactivated by radiation were reactivated and stimulated thus causing a thickening of individual hairs (close to the subject's anagen hair), a densification of the hair in the area of administration and even the appearance of weak hair in the central region of the head (the most irradiated area). For these reasons, this technique is also an effective and simple alternative for the treatment of this type of alopecia for which the only alternative is invasive surgery.

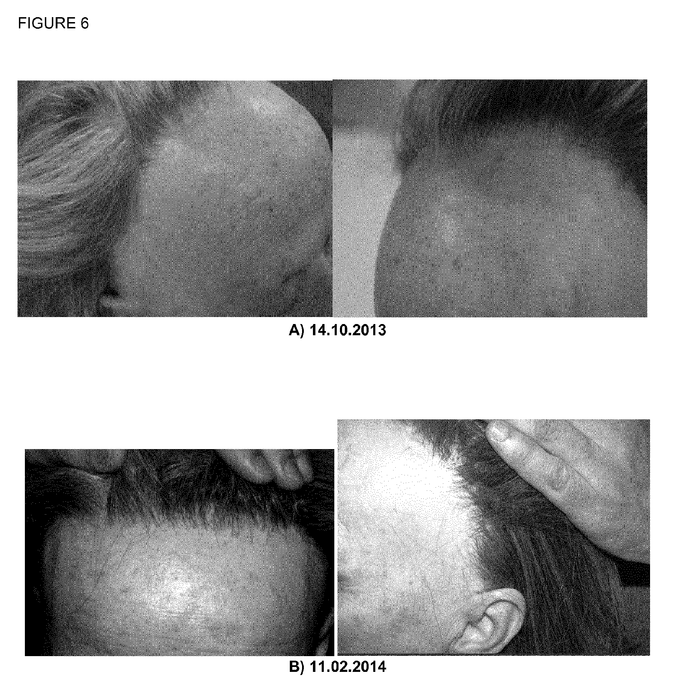

Moreover, the authors of the present invention have used the cell composition of the invention in the treatment of scarring frontal fibrosing alopecia. For this purpose, the composition product of Step 2 of example 3 (infiltration throughout the frontal region) was administered via subepidermally to a human subject suffering from this disease. As illustrated in table XI and also in figure 6, after treatment, the subject did not show any adverse effects and surprisingly, after four months from the first treatment, the composition suppressed the follicular inflammatory process and activated the follicular cells to stop the progression of alopecia. Therefore, this treatment clearly limited the spread of the alopecia. For these reasons, this technique is an effective and simple alternative for the treatment of this type of alopecia for which despite the existence of several drugs indicated for this disease, until now there has been no effective treatment. In fact the only successful treatment until now is capillary transplant, but this treatment is only successful provided that the disease has been stable for over 4 years and shows no new signs of active inflammation.

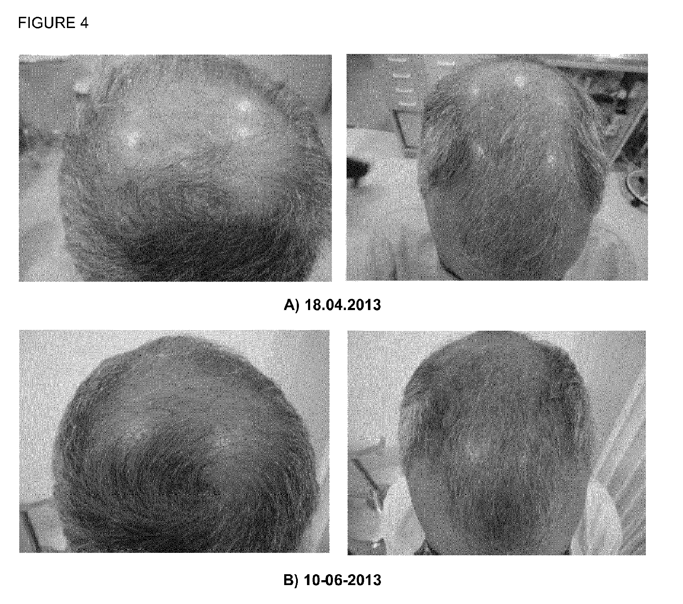

In addition, the authors of the present invention have used the cell composition of the invention in the treatment of non-scarring follicular miniaturization alopecia. In this case, the composition product of Step 2 of example 3 (infiltration throughout the frontal and vertex regions) was administered via subepidermally to a human subject suffering from this disease. As illustrated in table VIII below and also in figure 4, the subject did not show any adverse effects and surprisingly after three months from the first treatment, new dark hair appeared and centripetal hair was observed in the vertex and border with the frontal area. In addition, after four months from the first treatment, no new hair regression was observed. For these reasons, this technique is an effective and simple alternative for the treatment of this type of alopecia for which the only alternative is capillary transplant by surgery; of course this alternative is only feasible provided that there are populated donor areas in the occipital region of the head.

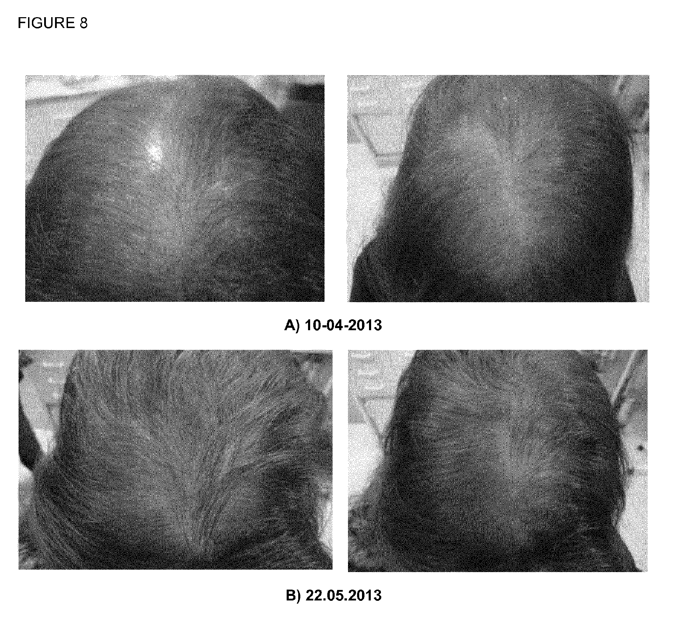

Finally, the authors of the present invention have used the cell composition of the invention in the treatment of effluviums. For this purpose, the composition product of Step 2 of example 3 (infiltration throughout the middle region) was administered via subepidermally to a human subject suffering from this disease. As illustrated in table XIII below and also in figure 8, after treatment, the subject did not show any adverse effects and surprisingly, after a follow-up period of 5 months the composition stabilized the hair loss and recovered hair density. For these reasons, this technique is an effective and simple alternative for the treatment of this type of alopecia for which no treatment has been demonstrated to be effective.

In conclusion, the cell suspension of the invention is capable of providing a progressive regulation and biological renewal of the follicular cells in the treated and surrounding areas, causing a perifollicular inflammation reduction, a follicular cycle miniaturization reduction, an increased in the number of anagen hairs and an increase in the activity of reserve cells that were inactive. Consequently, this cell suspension further comprising plasma derived growth factors, constitutes an effective and simple alternative for the treatment of all types of alopecia in which usual treatments are ineffective or for which the only alternative is invasive surgery.

Many methods are known in the art for the preparation of the composition comprising the cell suspension and the plasma derived growth factors referred to above. For the preparation of the plasma derived growth factors, different authors have described specific processes for obtaining the same. These procedures are based on the separation of the blood's cellular fraction from the plasma to form the clot which in turn contains the growth factors. Specific processes for obtaining the plasma derived growth factors are described in: Role of platelets and fibrin in the healing sequence: an in vivo study of angiogenesis and collagen synthesis. Knighton DR, Hunt TK, Thakral KK, Goodson WH 3rd. Ann Surg. 1982 Oct;196(4):379-88; Platelet-Rich Plasma: Properties and clinical applications. Rick G. Smith, B.S., C.C.P., Craig J. Glassmann, C.C.P., and Mark S. Campbell, B.A., C.C.P. Summer 2007 - Vol.2, No.2; Platelet-rich plasma: evidence to support its use. Marx RE. J Oral Maxillofac Surg. 2004 Apr;62(4):489-96; Platelet-rich plasma: Growth factor enhancement for bone grafts. Marx RE, Carlson ER, Eichstaedt RM, Schimmele SR, Strauss JE, Georgeff KR. Oral Surg Oral Med Oral Pathol Oral Radiol Endod. 1998 Jun;85(6):638-46; Platelet-derived growth factor is a chemoattractant for vascular smooth muscle cells. Grotendorst GR, Chang T, Seppä HE, Kleinman HK, Martin GR. J Cell Physiol. 1982 Nov;113(2):261-6; Identification of platelet proteins separated by twodimensional gel electrophoresis and analyzed by matrix assisted laser desorption/ionization-time of flight-mass spectrometry and detection of tyrosinephosphorylated proteins. Marcus K, Immler D, Sternberger J, Meyer HE. Electrophoresis. 2000 Jul;21(13):2622-36 and in Platelets and inflammation. Klinger MH. Anat Embryol (Berl). 1997 Jul;196(1):1-11. Therefore, the skilled person would surely be aware of a process to obtain the plasma derived growth factors cited through-out the present invention. As regards the cell suspension, the inventors have developed an effective method of preparing said cell suspension comprising HPCs from a human subject (suspension of the second aspect of the invention). Thus, a second aspect of the invention refers to a method of obtaining a cell suspension comprising HPCs from a human subject which comprises the following steps:
a. Obtaining a tissue sample comprising at least one complete hair follicle from the subject, preferably from the occipital region of the head of the subject;
b. Enzymatically or mechanically digest the cells from the tissue obtained from the sample of step a);
c. If needed, inactivate the enzyme of step b); and
d. Removing the tissue fragments from the cell suspension obtained from steps b) or c), preferably by filtration.

A preferred embodiment of the second aspect of the invention refers to a method of obtaining a cell suspension comprising HPCs from a human subject which comprises the following steps:
a. Obtaining a tissue sample comprising at least one complete hair follicle from the subject, preferably from the occipital region of the head of the subject;
b. Introducing the tissue obtained from step a) in a tube containing collagenase;
c. Incubating the tissue with collagenase for a period of time between 15 and 60 minutes while shaking at a temperature of approximately 37 °C;
d. Inactivating the enzyme of step c); and
e. Removing the tissue fragments from the cell suspension obtained from step d), preferably by filtration.

A preferred embodiment of the second aspect of the invention refers to a method of obtaining a cell suspension comprising HPCs from a human subject which comprises the following steps:
a. Obtaining a tissue sample comprising at least one complete hair follicle from the subject, preferably from the occipital region of the head of the subject;
b. Introducing the tissue in a tube containing 0.075 % w/v of collagenase;
c. Incubating the tissue with the collagenase for a period of time between 15 and 60 minutes while shaking, preferably for a period of approximately 30 minutes, at a temperature of approximately 37 °C;
d. Inactivating the enzyme of step c), preferably with human serum;
e. Removing the tissue fragments from the product of step d); and
f. Centrifuging the product of step e) and removing the supernatant.

A third aspect of the invention refers to a method of obtaining a composition comprising a cell suspension comprising HPCs from a human subject and plasma derived growth factors which comprises the following steps:
a. Obtaining a tissue sample comprising at least one complete hair follicle from the subject, preferably from the occipital region of the head of the subject;
b. Introducing the tissue obtained from step a) in a tube containing an enzyme suitable for the enzymatic digestion of the cells;
c. Incubating the tissue with the enzyme;
d. Inactivating the enzyme of step c);
e. Removing the tissue fragments from the product of step d);
f. Re-suspending the cell composition of step e) in a suspension of plasma derived growth factors, preferably obtained from the subject; and
g. Filtrating the cell suspension obtained from step f).

A preferred embodiment of the third aspect of the invention refers to a method of obtaining a composition comprising a cell suspension comprising HPCs from a human subject and plasma derived growth factors, which comprises the following steps:
a. Obtaining a tissue sample comprising at least one complete hair follicle from the subject, preferably from the occipital region of the head of the subject;
b. Introducing the tissue in a tube containing 0.075 % w/v collagenase;
c. Incubating the tissue with the collagenase for a period of time between 15 and 60 minutes while shaking at a temperature of approximately 37 °C, preferably for a period of approximately 30 minutes;
d. Inactivating the enzyme of step c), preferably with human serum;
e. Removing the tissue fragments from the product of step d);
f. Centrifuging the product of step e) and removing the supernatant;
g. Re-suspending the cell composition of step f) in a suspension of plasma derived growth factors, preferably obtained from the subject; and
h. Filtrating the cell suspension obtained of step g).

A fourth aspect of the invention refers to a method of obtaining a composition comprising a cell suspension comprising HPCs from a human subject and plasma derived growth factors which comprises the following steps:
a. Obtaining a tissue sample comprising at least one complete hair follicle from the subject, preferably from the occipital region of the head of the subject;
b. Incubating the tissue in a suspension of plasma derived growth factors, preferably obtained from the subject; for a period of time between 15 minutes and 1 hour at a temperature of approximately 37 °C;
c. Removing the tissue fragments from the product of step b); preferably by filtration.

A preferred embodiment of the fourth aspect of the invention refers to a method of obtaining a composition comprising a cell suspension comprising HPCs from a human subject and plasma derived growth factors, which comprises the following steps:
a. Obtaining a tissue sample comprising at least one complete hair follicle from the subject, preferably from the occipital region of the head of the subject;
b. Optionally, washing the tissue obtained from step a);
c. Incubating the tissue in a suspension of plasma derived growth factors, preferably obtained from the subject; for a period of time between 15 minutes and 1 hour at a temperature of 37 °C;
d. Homogenizing the cell composition of step c) by means of a mechanical or manual homogenizer such as a Potter homogenizer; and
e. Removing the tissue fragments from the product of step d); preferably by filtration.

A preferred embodiment of the fourth aspect of the invention refers to a method of obtaining a composition comprising a cell suspension comprising HPCs from a human subject and plasma derived growth factors, which comprises the following steps:
a. Obtaining a tissue sample comprising at least one complete hair follicle from the subject, preferably from the occipital region of the head of the subject;
b. Optionally, washing the tissue obtained from step a), preferably the washing step is done more than one time;
c. Incubating the tissue in a suspension of plasma derived growth factors, preferably obtained from the subject; for a period of time between 15 minutes and 1 hour at a temperature of approximately 37 °C;
d. Homogenizing the cell composition of step c) by means of a mechanical or manual homogenizer such as a Potter homogenizer; and
e. Removing the tissue fragments from the product of step d); preferably by filtration.

A preferred embodiment of the fourth aspect of the invention refers to a method of obtaining a composition comprising a cell suspension comprising HPCs from a human subject and plasma derived growth factors, which comprises the following steps
a. Obtaining a tissue sample comprising at least one complete hair follicle from the subject, preferably from the occipital region of the head of the subject;
b. Optionally, washing the tissue obtained from step a), preferably this washing step is done more than one time;
c. Incubating the tissue in a suspension of plasma derived growth factors, preferably obtained from the subject; for a period of time of approximately 30 minutes at a temperature of approximately 37 °C;
d. Homogenizing the cell composition of step c) by means of a mechanical or manual homogenizer such as a Potter homogenizer; and
e. Removing the tissue fragments from the product of step d); preferably by filtration.

A fifth aspect of the invention refers to a cell suspension comprising hair progenitor cells (HPCs) of a human subject obtained or obtainable by means of the method of the second aspect of the invention.

A sixth aspect of the invention refers to a composition comprising a cell suspension comprising HPCs from a human subject and plasma derived growth factors preferably obtained from the said human subject, obtained or obtainable by means of the method of the third aspect of the invention.

A seventh aspect of the invention refers to a composition comprising a cell suspension comprising HPCs from a human subject and plasma derived growth factors preferably obtained from the said human subject, obtained or obtainable by means of the method of the fourth aspect of the invention.

In an eight aspect of the present invention, the invention provides methods of treating hair loss, treating, inhibiting, or suppressing a degenerative skin disorder, and treating scarring and non-scarring alopecia, such as androgenetic alopecia (AGA), in a subject and generating new hair follicles (HF) and increasing the size of existing HF, comprising the administration of the cell suspension or composition of any of aspects fifth, sixth or seventh. Thus, in one preferred embodiment of the eight aspect of the invention, the present invention provides a method of treating hair loss in a subject comprising the steps of (a) obtaining the composition or the cell suspension of any of aspects fifth, sixth or seventh and (b) administering said composition or cell suspension to the subject. In a preferred embodiment, the administering step is via subepidermal, intralesional, dermal or epidermal.

In one embodiment of the eight aspect of the invention, the hair loss is due to androgenetic alopecia (AGA). In one embodiment of the eight aspect of the invention, the AGA is male pattern baldness. In another embodiment, the AGA is female pattern baldness. In one embodiment of the eight aspect of the invention, the hair loss is the result of a skin injury. In one embodiment of the eight aspect of the invention, the hair loss is in the scalp or eyebrow of said subject. In one embodiment of the eight aspect of the invention, the hair loss is in scarred skin tissue of said subject. In one embodiment of the eight aspect of the invention, the scarring alopecia is hair implant scarring alopecia. In one embodiment of the eight aspect of the invention, the scarring alopecia is acquired by radiation. In one embodiment of the eight aspect of the invention, the scarring alopecia is frontal fibrosing scarring alopecia. In one embodiment of the eight aspect of the invention, the hair loss is the result of a non-scarring alopecia. In one embodiment of the eight aspect of the invention, the non-scarring alopecia is autoimmune non-scarring (Areata) alopecia. In one embodiment of the eight aspect of the invention, the non-scarring alopecia is non-scarring follicular miniaturization alopecia. In one embodiment of the eight aspect of the invention, the non-scarring alopecia is Effluviums.

In another embodiment of the eight aspect of the invention, the present invention provides a method for generating a hair follicle in the skin of a subject with hair loss comprising the steps of (a) obtaining the composition or cell suspension of any of aspects fifth, sixth or seventh and (b) administering the same to the subject. In a preferred embodiment, the administering step is via subepidermal, intralesional, dermal or epidermal. In another embodiment of the eight aspect of the invention, the present invention provides a method for increasing the size of a hair follicle in the skin of a subject with hair loss comprising the steps of (a) obtaining the composition or cell suspension of any of aspects fifth, sixth or seventh and (b) administering the same to the subject. In a preferred embodiment, the administering step is via subepidermal, intralesional, dermal or epidermal. In another embodiment, the present invention provides a method for increasing hair follicle formation in the skin of a subject with hair loss comprising the steps of (a) obtaining the composition or cell suspension of any of aspects fifth, sixth or seventh and (b) administering the same to the subject. In a preferred embodiment, the administering step is via subepidermal, intralesional, dermal or epidermal. In another embodiment, the present invention provides a method for treating, inhibiting, or suppressing a degenerative skin disorder comprising the steps of (a) obtaining the composition or cell suspension of any of aspects fifth, sixth or seventh and (b) administering the composition to the subject. In a preferred embodiment, the administering step is via subepidermal, intralesional, dermal or epidermal.

In a further embodiment, the cell suspensions of the invention may be implanted or injected into the subject together with a matrix forming component. This may allow the cells to form a matrix following injection or implantation, ensuring that the cells remain at the appropriate location within the subject. Examples of matrix forming components include fibrin glue liquid alkyl, cyanoacrylate monomers, plasticizers, polysaccharides such as dextran, ethylene oxide-containing oligomers, block co-polymers such as poloxamer and Pluronics, nonionic surfactants such as Tween and Triton'8', and artificial matrix forming components. This list is provided by way of illustration only, and is not intended to be limiting. It will be clear to a person skilled in the art, that any combination of one or more matrix forming components may be used.

In another specific embodiment, the cell suspensions of the invention may be frozen in freezing medium. Any medium that preserves the viability of the cells at temperatures below about -20 °C (e.g. temperatures below about -40 °C, or about -80 °C, or about -190 °C is suitable as freezing medium. For example, the freezing medium may comprise 2.5 % to 10 % DMSO. More specifically, the freezing medium may comprise 5-7.5 % DMSO.

After thawing, cells of invention can be washed to remove the DMSO or other freezing medium components before administration or re-suspension in administration solution. Administration solution will be any physiological solution able to be injected in patients without toxicity, in the present case the administration solution would preferably be the plasma derived growth factors.

Lastly, for use in therapy and methods of treatment, the cell suspensions of the invention will be delivered to the subject in a therapeutically effective amount. The number of cells to be delivered *in vivo* is based on a number of parameters, including: the body weight of the subject, the severity of tissue damage, and the number of cells surviving within the subject.

The purpose of the following examples is merely to illustrate the present invention.

### EXAMPLES

### Example 1. Materials and methods

### 1.1. Tissue samples

The biopsies (tissue punches or tissue samples) used to illustrate the present invention were obtained in an operating room, wherein the processing of the biopsies as well as the processes of obtaining the blood plasma-derived growth factors were performed in a clean room with classification C (ISO 7), thus complying with the regulation in force in Spain.

### 1.2. Quality controls

Given that these quality controls are for autologous treatments, a series of quality controls were carried out that allowed the assurance of sample traceability, as well as avoiding any type of microbiological contamination and cross contamination with other products. The controls that were performed prior to taking the biopsies were as follows:

### • Serology

Objective: quality control of the blood sample to prevent workers from getting infected.

Procedure: Blood is drawn days before obtaining the HPCs in a clinical analysis. This blood sample is used to determine the existence or absence of infectious diseases such as HIV, Hepatitis B and C and *Treponema pallidum.*

If the patient is free of these diseases, the procedure will continue. Otherwise, the human subject will be rejected.

### • Obtaining a saliva sample and several hairs comprising the root of the hair.

Objective: to control traceability of the samples by means of genetic identification.

### Procedure for taking saliva sample:

a. Used individual wrapped sterile oral swabs;
b. Remove swab from its wrapper with clean hands and prevent the cotton end of the swab from contacting any element (fingers, face, objects, etc.);
c. Introducing the cotton of the swab in the mouth of the subject;
d. Rubbing the cotton of the swab for 30 seconds inside the mouth (cheeks, behind the lips and under the tongue);
e. Allowing the cotton to dry without it touching any object;
f. Introducing the swab in an Eppendorf tube and cut the stick; and
g. Storing the perfectly labelled cotton of the swab at -20 °C.

### Procedure for taking a hair sample:

It is done at the same time that the tissue samples are obtained from the head of the subject and comprises the following steps:
a. Pulling several hairs from the patient using Adson forceps taking into account the fact that if the hairs do not have a root, they are disposed of and others are taken;
b. Introduce the hairs in an Eppendorf tube; and
c. Store the perfectly labelled tube at -20 °C.

They are finally stored for the purpose of being able to perform the necessary examinations in the event of adverse events or doubts as to the traceability.

### • Reference samples

Objective: to control sample traceability by means of genetic identification. It will be used as a reference sample to be compared with the saliva and hair samples.

### Example 2. Isolation method of hair progenitor cells (HPCs) from a human subject by enzymatic digestion (Type I collagenase)

### Step 1: Obtaining the growth factors (GFs)

a. Drawing 4 citrate tubes of blood in an operating room from the subject;
b. Centrifuging the 4 tubes of blood with citrate for 10 minutes at 4000 rpm at room temperature in a clean room;
c. Collecting the plasma after centrifugation with a micropipette (preferably collect the fraction of plasma closest to the erythrocyte fraction, the remaining blood components are discarded);
d. Adding calcium chloride: (24 µl of 10% CaCl2 for every 1 ml of plasma) to the plasma obtained in step c);
e. Incubating the product of step d) in a steaming cabinet at 37 °C from 1 to 24 hours until a coagulum is obtained;
f. Once the clot is formed, this is introduced into a petri dish in a laminar flow bio IIA. The clot is left on a petri dish and pressure is applied on the petri dish in order to accelerate the growth factor secretion process. The factors once secreted are collected by using a micropipette and introduce into a new sterile tube, the clot is discarded once the growth factors are obtained.

### Step 2: Obtaining the hair progenitor cells (HPCs).

a. Obtaining approximately 10 punches of 1.5 mm of diameter, from the occipital area of the head of a human subject;
b. Adding the punches to a tube containing 1 ml of sterile 1X PBS;
c. Centrifuging the product of step b) for 5 minutes at 1.200 rpm at room temperature;
d. Removing the supernatant of step c) and cutting the punches into fragments using a disposable scalpel;
e. Introducing the tissue of step d) in a tube containing 1 ml 0.075 % w/v collagenase (previously activated at 37 °C for 30 minutes);
f. Incubating the product of step e) at 37 °C for 30 minutes while shaking;
g. Inactivating the collagenase with 100 µl of 10 % human serum;
h. Removing the tissue fragments from the product of step g);
i. Centrifuging the cell suspension of step h) for 10 minutes at 1200 rpm at room temperature;
j. Removing the supernatant from step i) and re-suspending the cell composition obtained, in the suspension of growth factors illustrated in step 1 above, wherein the suspension of growth factors derived from plasma is preferably obtained from the same patient and wherein preferably the cell composition is re-suspended in 0.5 to 10 ml of the suspension of growth factors derived from plasma and more preferably the cell composition is re-suspended in 2 to 4 ml of the suspension of growth factors derived from plasma;
k. Filtering the suspension through a 40 µm cell strainer;
l. Quantifying the cells of the obtained suspension by means of a manual counter using the trypan blue exclusion method, thus determining the number of cells and their viability;
m. Inoculating the entire cell suspension obtained (number. depending on the patient) aliquoted into 4 (insulin-type) syringes.

The method of trypan blue exclusion is based on the ability of the dye trypan blue to exclusively stain dead cells. Thus under a microscope it is easy to differentiate live cells from dead cells, the latter comprise a blue colour.

Please note that the authors of the invention have used an incubation time of 30 minutes in step f). The reason for the election of this specific incubation time period is not an arbitrary selection as illustrated in the tables below.

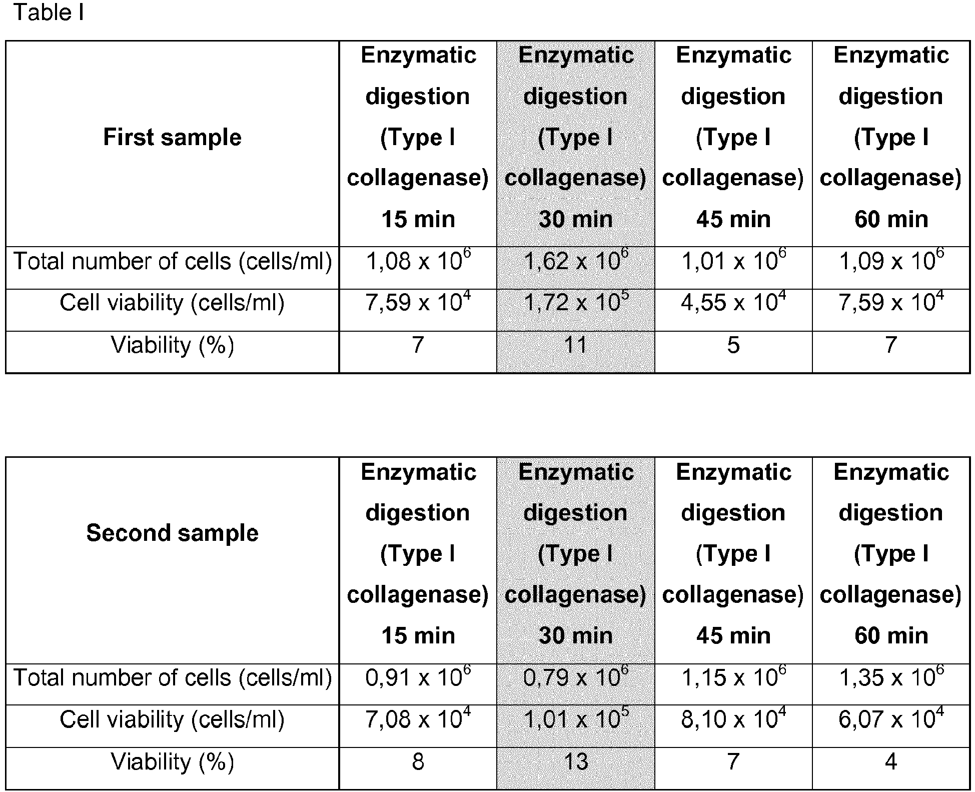

### Example 3. Isolation method of hair progenitor cells (HPCs) from a human subject by mechanical digestion

### Step 1: Obtaining the growth factors (GFs)

a. Drawing 4 citrate tubes of blood in an operating room from the subject;
b. Centrifuging the 4 tubes of blood with citrate for 10 minutes at 4000 rpm at room temperature in a clean room;
c. Collecting the plasma after centrifugation with a micropipette (preferably collect the fraction of plasma closest to the erythrocyte fraction, the remaining blood components are discarded);
d. Adding calcium chloride: (24 µl of 10 % CaCl2 for every 1 ml of plasma) to the plasma obtained in step c);
e. Incubating the product of step d) in a steaming cabinet at 37 °C from 1 to 24 hours until a coagulum is obtained;
f. Once the clot is formed, this is introduced into a petri dish in a laminar flow bio IIA. The clot is left on a petri dish and pressure is applied on the petri dish in order to accelerate the growth factors secretion process. The factors once secreted are collected by using a micropipette and introduced into a new sterile tube; the clot is discarded once the growth factors are obtained.

### Step 2: Obtaining the hair progenitor cells (HPCs).

a. Obtaining at most 10, 1.5 mm, punches from the occipital area of the head of a human subject;
b. Adding the punches obtained from step a) to a tube containing 1 ml of sterile 1X PBS;
c. Centrifuging for 5 minutes at 1.200 rpm at room temperature the product of step b);
d. Removing the supernatant of step c) and washing again with 1X PBS like in b) above;
e. Centrifuging the product of step d) for 5 minutes at 1200 rpm at room temperature;
f. Removing the supernatant from the product of step e) and cutting the punches into fragments using a disposable scalpel;
g. Incubating the tissue of step f) at 37 °C with constant gentle stirring in 1 ml of the autologous growth factors obtained in step 1 above for aproximately 30 minutes;
h. After incubation, homogenizing the tissue of step g) by means of a homogenizer (manual or mechanical homogenizer);
i. Filtering the product of step h) with a 40 µm cell strainer;
j. Collecting the filtered supernatant and performing the cell count using the trypan blue exclusion technique;
k. Aliquoting the product into 4, 1 ml (insulin-type) syringes for administration.

The authors of the present invention conducted two different experiments to determine the differences, if any, in terms of viability and total number of cells obtained by using the isolation method of HPCs by means of an enzymatic digestion as illustrated in example 2 above and the isolation method by means of a mechanical digestion as illustrated in example 3. To compare both methods, quantification was performed by the method of exclusion by trypan blue automatically. For this purpose the TC20 equipment of Bio-Rad was used.

For the first experiment (Table II below) both isolation methods were conducted by using different 1.5 mm punches obtained from the occipital area of the head of a human subject. Surprisingly, the isolation method by means of a mechanical digestion (example 3) provides a significantly larger total number of cells in addition to a greater percentage of viability of these cells. This result was completely unexpected making the isolation method by means of a mechanical process a simpler and more efficient process to obtain the cell product of the invention. This first experiment was repeated by using different 1.5 mm punches obtained from the occipital area of the head of a second human subject. The results obtained were similar to the ones obtained in the first experiment and illustrate the advantages of using the isolation process described in example 3.

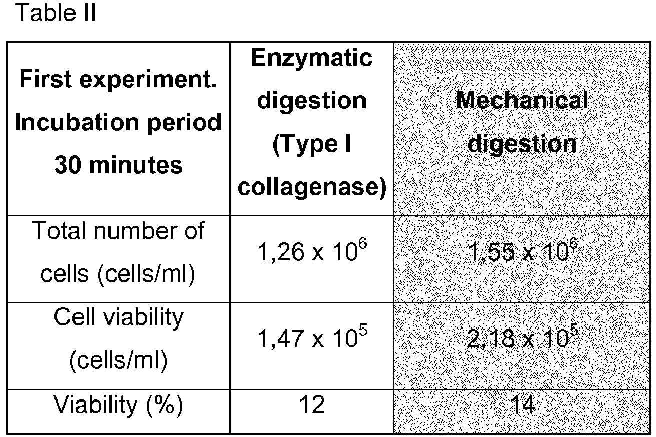

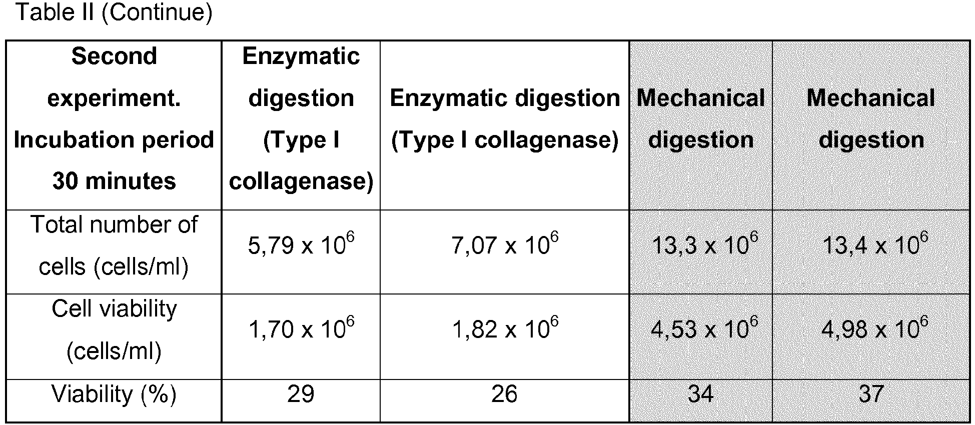

### Example 4. Method of treatment of scarring alopecia acquired by hair implant

The authors of the present invention evaluated the efficacy of the cell composition of the invention in the treatment of scarring alopecia acquired by hair implant. In this sense, they administered (Infiltration throughout the frontal, parietal and vertex regions) the product of Step 2 of example 3 to a human subject suffering from scarring alopecia acquired by hair implant. As illustrated in tables III and IV below and also in figure 1, the administration of this product causes a progressive regulation and biological renewal in the treated area and surrounding areas, in fact it causes an increased in the number of anagen hairs and an increased in the activity of reserve cells inactive until now. For these reasons, this technique is an effective and simple alternative for the treatment of scarring alopecia acquired by hair implant for which the only alternative is surgical hair transplant; however this type of transplant is only possible if the subject has a populated donor area in the occipital region of the head.

**Table III**

| **PATIENT JHM1978 (TREATMENT WITH CELLS)** | | | | | | | | | | | | |
|---|---|---|---|---|---|---|---|---|---|---|---|---|
| INDICATION | PATIENT CODE | SEX | AGE | TYPE OF ALOPECIA | DISEASES | ADVANCED MEDICAL TREATMENT DATE | DOSE | TOTAL CELL COUNT | VOLUME OF SUSPENSION TO INJECT | AREA TREATED (CM²) | LOCATION OF IMPLANT | GF INFILTRATION |
| | | | | | | | | | | | | |
| Scarring alopecia acquired by hair implant | JHM1978 | M | 35 | Acquired scarring | no | 29.01.2013 | 1^{st} | 670,000 | 4 ml | 144 | frontal, parietal and vertex | about every 15 days |
| | | | | | | 25.05.2013 | 2^{nd} | 1,500,000 | 4 ml | 144 | frontal, parietal and vertex | about every 15 days |
| | | | | | | 14.11.2013 | 3rd | 2,500,000 | 3.5 ml | 144 | frontal, parietal and vertex | about every 15 days |

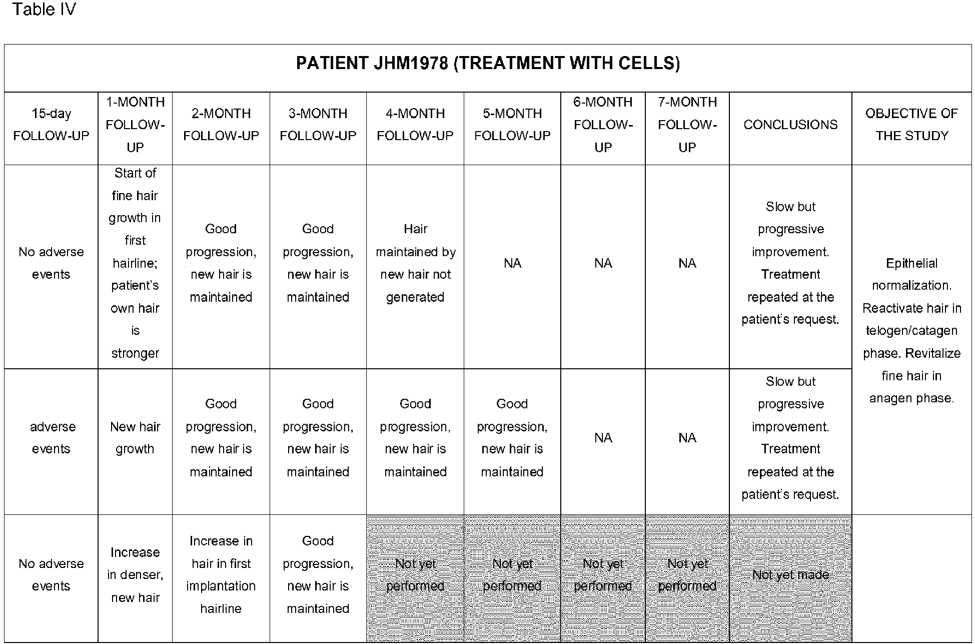

### Example 5. Method of treatment of non-scarring autoimmune (Areata) alopecia

Current medical treatments for non-scarring autoimmune (Areata) alopecia are based on the following pharmaceutical compositions:
o Corticosteroids: as a result of their anti-inflammatory and immunosuppressive effect; during treatment hair grows back but once treatment is suppressed hair loss appears again.
   ■ Contraindications of topical corticosteroids: causes erythema, acneiform eruption, stretch marks, telangiectasia, demodicidosis, hypertrichosis, hypopigmentation, milium cysts, purpura, stellate pseudoscars, tachyphylaxis and dermatophytosis.
   ■ Contraindications of systemic and intralesional corticosteroids: pseudo-Cushing's, suppression of the hypothalamus-pituitary-renal axis, osteonecrosis of femoral head, growth inhibition, cataracts, night sweats, edema, weight loss or gain, headache and amaurosis due to thrombosis of the central retinal artery.
∘ Anthralin as a result of its immunomodulatory effect; it inhibits cytotoxic activity and IL-2 production and normalizes the suppressor T-cell function
   ■ Contraindications: change in hair color, intense pruritus, regional adenomegalies and localized bacterial superinfection.
∘ Minoxidil because it is believed that it prolongs the anagen phase, it is used in association with topical corticoids or anthralin. However, there are no studies that demonstrate it.
   ■ Contraindications: localized facial hypertrichosis.
∘ Systemic cyclosporin acts like an immunosuppressant at the CD4 T-cell level.
   ■ Contraindications: blood pressure, creatinine, and liver function must be monitored. It can cause gingival hyperplasia, hypertrichosis and sebaceous hyperplasia.
∘ Topical immunotherapy by means of sensitizing the patient with a laboratory allergen that is subsequently applied in a bald area causing an eczematous reaction which leads to an inflammatory infiltrate and shifts the specific lymphocyte infiltrate of alopecia.
   ■ Contraindications: depends on the allergen used, risk of carcinogenesis, contact dermatitis, development of adenomegalies, pruritus, vesicles, blisters, urticaria, multiforme erythema, vitiligo
∘ Immunomodulators such as biotin, SDZ ASM981, mycophenolic acid, pyridoxine, pantothenic acid, zinc sulfate. There are not very well known and there are few studies on its effects.

Therefore, to date there is no effective treatment of this type of alopecia that is not accompanied by adverse effects.

Thus, in order to evaluate the efficacy and safety of the cell composition of the invention in the treatment of non-scarring autoimmune (Areata) alopecia, the authors of the invention administered via subepidermal to a patient suffering from this disease the composition product of Step 2 of example 3. As illustrated in table V and also in figure 2, the administration of this product did not produce any adverse effects and after one month of its first administration new hair progressively appeared. In addition, after two months of the first administration increasingly stronger hair was observed in the different regions and surrounding areas were the product was originally applied. For these reasons, this technique is an effective and simple alternative for the treatment of this type of alopecia for which the current alternatives have shown a great number of contraindications as well as relatively mild effects in the treatment of hair loss caused by this type of alopecia.

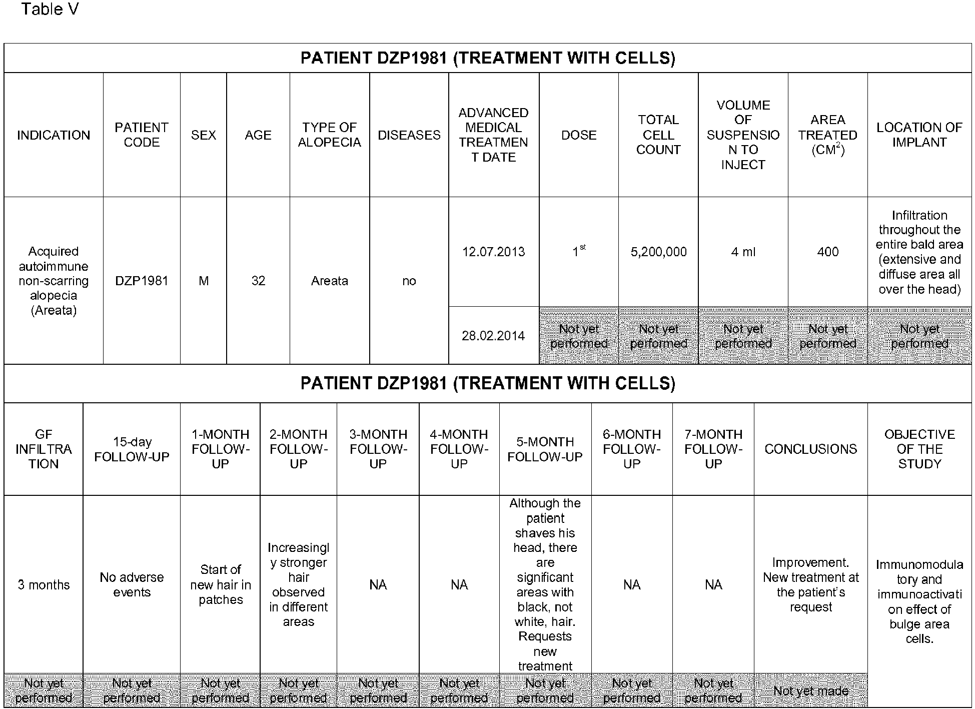

### Example 6. Method of treatment of scarring alopecia acquired by radiation

In order to evaluate the efficacy of the cell composition of the invention in the treatment of scarring alopecia acquired by radiation, the authors of the invention administered via subepidermal (Infiltration throughout the entire bald area (extensive and diffuse area all over the head)) to a human subject suffering from this disease, the composition product of Step 2 of example 3. As illustrated in tables VI and VII below and also in figure 3, the human subject did not show any adverse effects and surprisingly after two months from the first administration of the composition product of the invention, the cells of the hair follicles inactivated by radiation were reactivated and stimulated thus causing a thickening of individual hairs (close to the subject's anagen hair), a densification of the hair in the area of administration and even the appearance of weak hair in the central region of the head (the most irradiated area). For these reasons, this technique is an effective and simple alternative for the treatment of this type of alopecia for which the only alternative is invasive surgery.

**Table VI**

| **PATIENT IIG1982 (TREATMENT WITH CELLS)** | | | | | | | | | | | | |
|---|---|---|---|---|---|---|---|---|---|---|---|---|
| INDICATION | PATIENT CODE | SEX | AGE | TYPE OF ALOPECIA | DISEASES | ADVANCED MEDICAL TREATMENT DATE | DOSE | TOTAL CELL COUNT | VOLUME OF SUSPENSION TO INJECT | AREA TREATED (CM²) | LOCATION OF IMPLANT | GF INFILTRATION |
| Scarring alopecia acquired by radiation | IIG1982 | F | 31 | Caused by radiation | Grade II astrocyto ma (treatme nt at 11 years of age) | 23.05.2013 | 1^{st} | 1,200,000 | 6 ml | 100 | Infiltration throughout the entire bald area (extensive and diffuse area all over the head) | 15 days, 2, 3, 4, and 5 months |
| | | | | | | 21.11.2013 | 2^{nd} | 5,240,000 | 3.5 ml | 100 | Infiltration throughout the entire bald area (extensive and diffuse area all over the head) | 1, 3 months |

**Table VII**

| **PATIENT IIG1982 (TREATMENT WITH CELLS)** | | | | | | | | | |
|---|---|---|---|---|---|---|---|---|---|
| 15-day FOLLOW-UP | 1-MONTH FOLLOW-UP | 2-MONTH FOLLOW-UP | 3-MONTH FOLLOW-UP | 4-MONTH FOLLOW-UP | 5-MONTH FOLLOW-UP | 6-MONTH FOLLOW-UP | 7-MONTH FOLLOW-UP | CONCLUSIONS | OBJECTIVE OF THE STUDY |
| No adverse events, light itching resulting from the injections. Normalcy, more and thicker hair | No adverse reactions. Patient happy. | Thickening of individual hairs (close to the patient's anagen hair), greater centripetal densification. | NA | NA | Stronger, less weak hair and general thickening of the hair | NA | NA | Hair stronger. New treatment at the patient's request | Reactivate and stimulate cells inactivated by radiation. Epithelial normalization. |
| No adverse events. | Hair stronger, densification of the area; onset of fine and weak hair in the central area (the most irradiated area). Patient very satisfied. | Progressing satisfactorily | NA | NA | An increase in length, the stem thickness and deeper color hair of the patient is observed. | Not yet performed | Not yet performed | Not yet made | |

### Example 7. Method of treatment of non-scarring follicular miniaturization alopecia

In order to evaluate the efficacy of the cell composition of the invention in the treatment of non-scarring follicular miniaturization alopecia, the composition product of Step 2 of example 3 (infiltration throughout the frontal and vertex regions) was administered via subepidermally to a human subject suffering from this disease. As illustrated in table VIII below and also in figure 4, the subject did not show any adverse effects and surprisingly after three months from the first treatment, new dark hair appeared and centripetal hair was observed in the vertex and border with the frontal area. In addition, after four months from the first treatment, no new hair regression was observed. For these reasons, this technique is an effective and simple alternative for the treatment of this type of alopecia for which the only alternative is capillary transplant by surgery; of course this alternative is only feasible provided that there are populated donor areas in the occipital region of the head.

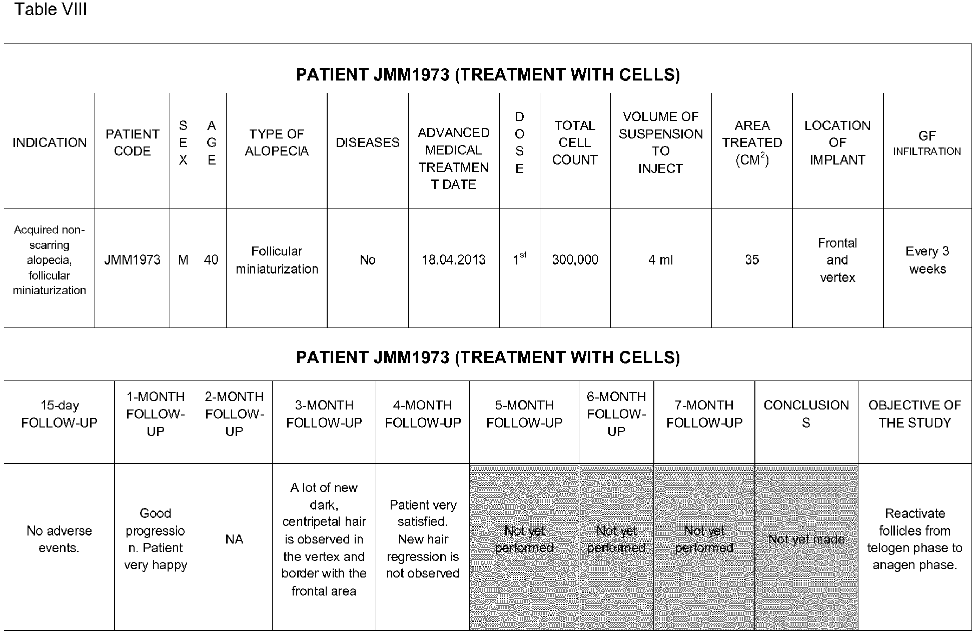

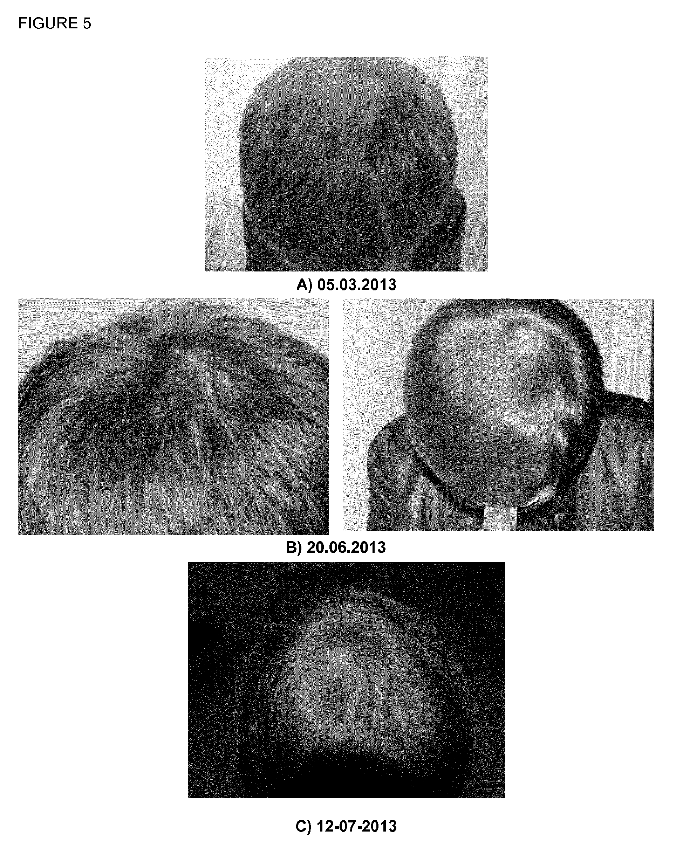

In addition, to further evaluate the efficacy of the cell composition of the invention in the treatment of non-scarring follicular miniaturization alopecia, the composition product of Step 2 of example 3 (infiltration throughout the frontal and vertex regions) was administered via subepidermally to a second human subject suffering from this disease. As illustrated in tables IX and X below and also in figure 5, the subject did not show any adverse effects and surprisingly the composition product of the invention produced a normalization of the seborrheic dermatitis suffered by the subject. In addition, after two months from the first treatment, a more densified area with strong hairs appeared in the infiltration region. For these reasons, this technique is an effective and simple alternative for the treatment of this type of alopecia for which the only alternative is capillary transplant by surgery; of course this alternative is only feasible provided that there are populated donor areas in the occipital region of the head.

**Table IX**

| **PATIENT ZBO1969 (TREATMENT WITH CELLS)** | | | | | | | | | | | | |
|---|---|---|---|---|---|---|---|---|---|---|---|---|
| INDICATION | PATIENT CODE | SEX | AGE | TYPE OF ALOPECIA | DISEASES | ADVANCED MEDICAL TREATMENT DATE | DOSE | TOTAL CELL COUNT | VOLUME OF SUSPENSION TO INJECT | AREA TREATE D (CM²) | LOCATION OF IMPLANT | GF INFILTRATION |
| Acquired non-scarring alopecia, follicular miniaturiza tion | ZBO 1969 | M | 44 | Follicular miniaturizati on | Psychiat ric disease | 05.03.2013 | 1^{st} | 630,000 | 4 ml | 100 | Frontal and vertex | Monthly |
| | | | | | | 21.05.2013 | 2^{nd} | 1,300,000 | 4 ml | 100 | Frontal and vertex | Monthly |

**Table X**

| **PATIENT ZBO1969 (TREATMENT WITH CELLS)** | | | | | | | | | |
|---|---|---|---|---|---|---|---|---|---|
| 15-day FOLLOW-UP | 1-MONTH FOLLOW-UP | 2-MONTH FOLLOW-UP | 3-MONTH FOLLOW-UP | 4-MONTH FOLLOW-UP | 5-MONTH FOLLOW-UP | 6-MONTH FOLLOW-UP | 7-MONTH FOLLOW-UP | CONCLUSIONS | OBJECTIVE OF THE STUDY |
| No adverse events. | Normalization of the seborrheic dermatitis. | Patient satisfied. Greater densification. | NA | NA | NA | NA | NA | Greater densification. Patient requests new treatment | Reactivate follicles from telogen phase to anagen phase. |
| No adverse events. | NA | More densified area with strong hairs and anagens. Itching stops. | NA | NA | NA | NA | NA | No itching. | |

### Example 8. Method of treatment of scarring frontal fibrosing alopecia

In order to evaluate the efficacy of the cell composition of the invention in the treatment of scarring frontal fibrosing alopecia, the composition product of Step 2 of example 3 (infiltration throughout the frontal region) was administered via subepidermally to a human subject suffering from this disease. It is noted that this type of alopecia must be treated to limit the spread of alopecia

As it is illustrated in table XI below and also in figure 6, after treatment, the subject did not show any adverse effects and surprisingly, after four months from the first treatment, the composition suppressed the follicular inflammatory process and activated the follicular cells to stop the progression of alopecia. Therefore, this treatment clearly limited the spread of alopecia. For these reasons, this technique is an effective and simple alternative for the treatment of this type of alopecia for which despite the existence of several drugs indicated for this disease, until now there has been no effective treatment. In fact the only successful treatment until now is capillary transplant, but this treatment is only successful provided that the disease has been stable for over 4 years and shows no new signs of active inflammation. In addition to this treatment, other usual treatments are:
o Corticoids provide benefit as a result of their anti-inflammatory effect but they can cause atrophy of the skin. It cannot be used in all steps of the disease. It can be applied topically, intralesionally or systemically.
∘ Anti-malaria drugs seem to bring about an improvement in erythema and follicular hyperkeratosis, as well as of the inflammatory signs of the disease, however it does not modify the course of the disease.
∘ Dutasteride is a 5-alpha reductase II inhibitor with an action that is similar to finasteride.
∘ Finasteride efficacy has not been demonstrated in post-menopausal women or in men over 41 years of age.

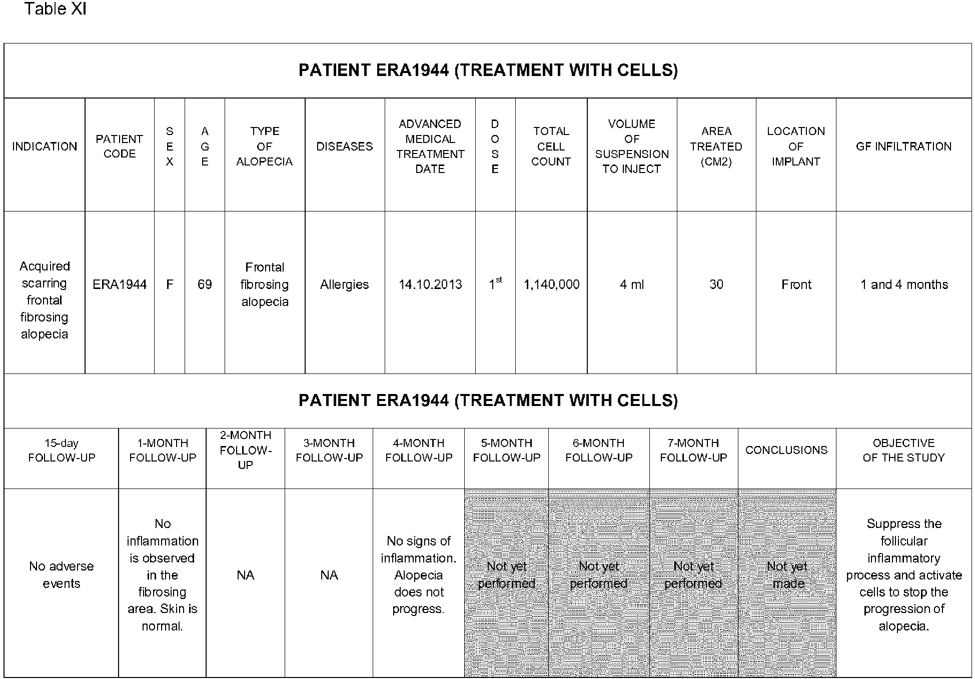

In addition, in order to evaluate the efficacy of the cell composition of the invention in the treatment of scarring frontal fibrosing alopecia, the composition product of Step 2 of example 3 (infiltration throughout the frontal region) was also administered via subepidermally to a second human subject suffering from this disease to determine whether this treatment could limit the spread of alopecia.

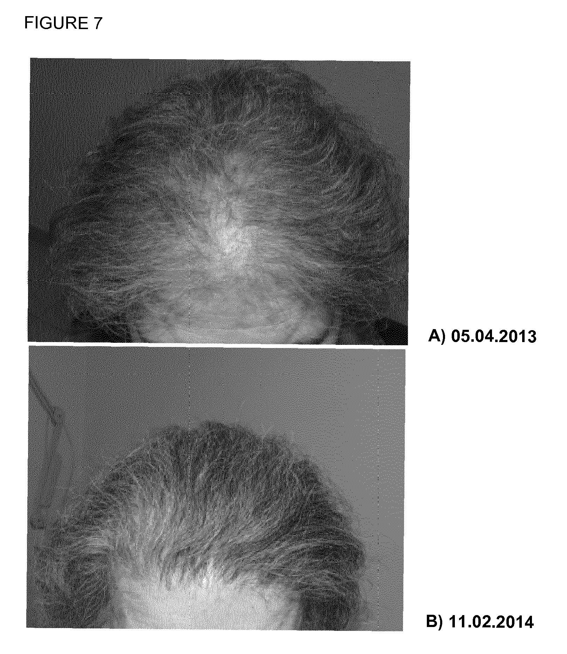

As illustrated in table XII below and also in figure 7, after treatment, the subject did not show any adverse effects and surprisingly, after three months the composition suppressed the follicular inflammatory process and activated the follicular cells to stop the progression of alopecia. Therefore, this proofs that this treatment limits the spread of alopecia and for this reason; this technique is an effective and simple alternative for the treatment of this type of alopecia for which despite the existence of several drugs indicated for this disease, until now there has been no effective treatment.

**Table XII**

| **PATIENT SFR1945 (TREATMENT WITH CELLS)** | | | | | | | | | | | | | | | | | | | |
|---|---|---|---|---|---|---|---|---|---|---|---|---|---|---|---|---|---|---|---|
| INDICATION | PATIENT CODE | | SEX | AGE | TYPE OF ALOPECIA | | DISEASES | | ADVANCED MEDICAL TREATMENT DATE | | DOSE | TOTAL CELL COUNT | | VOLUME OF SUSPENSION TO INJECT | | AREA TREATED (CM²) | | LOCATION OF IMPLANT | GF INFILTRATION |
| Acquired scarring frontal fibrosing alopecia | SFR1945 | | F | 68 | Frontal fibrosing alopecia | | Osteoporosis | | 09.07.2013 | | 1^{st} | 6,000,000 | | 4 ml | | 30 | | Frontal | 15 days, 1, 2, 3, 4, 5 and 6 months |

| **PATIENT SFR1945 (TREATMENT WITH CELLS)** | | | | | | | | | | | | | | | | | | | |
|---|---|---|---|---|---|---|---|---|---|---|---|---|---|---|---|---|---|---|---|
| 15-day FOLLOW-UP | | 1-MONTH FOLLOW-UP | | 2-MONTH FOLLOW-UP | | 3-MONTH FOLLOW-UP | | 4-MONTH FOLLOW-UP | | 5-MONTH FOLLOW-UP | | | 6-MONTH FOLLOW-UP | | 7-MONTH FOLLOW-UP | | CONCLUSIONS | | OBJECTIVE OF THE STUDY |
| No adverse events. Stronger and thicker hair observed. | | No adverse events. Stronger and thicker hair observed. | | Darker hair. | | No significant changes. Alopecia stabilizes. | | NA | | NA | | | NA | | NA | | Alopecia stabilizes. No more hair loss. | | Suppress the follicular inflammatory process and activate cells to stop the progression of alopecia. |

### Example 9. Method of treatment of Effluviums

In order to evaluate the efficacy of the cell composition of the invention in the treatment of effluviums, the composition product of Step 2 of example 3 (infiltration throughout the middle region) was administered via subepidermally to a human subject suffering from this disease.

As illustrated in table XIII below and also in figure 8, after treatment, the subject did not show any adverse effects and surprisingly, after a follow-up period of 5 months the composition stabilized the hair loss and recovered hair density. For these reasons, this technique is an effective and simple alternative for the treatment of this type of alopecia for which no treatment has been demonstrated to be effective.

**Table XIII**

| **PATIENT BUB1972 (TREATMENT WITH CELLS)** | | | | | | | | | | | | | | | | | | | |
|---|---|---|---|---|---|---|---|---|---|---|---|---|---|---|---|---|---|---|---|
| INDICATION | PATIENT CODE | SEX | | AGE | TYPE OF ALOPECIA | | DISEASES | | ADVANCED MEDICAL TREATMEN T DATE | | DOSE | TOTAL CELL COUNT | VOLUME OF SUSPENSION TO INJECT | | AREA TREATED (CM²) | | LOCATION OF IMPLANT | | GF INFILTRATION |
| Acquired non-scarring alopecia. Effluvium. | BUB1972 | F | | 41 | Effluvium | | No | | 10.04.2013 | | 1^{st} | 500,000 | 4 ml | | 49 | | Middle area | | 15 days, 1, 2, 3 months |

| **PATIENT BUB1972 (TREATMENT WITH CELLS)** | | | | | | | | | | | | | | | | | | | |
|---|---|---|---|---|---|---|---|---|---|---|---|---|---|---|---|---|---|---|---|
| 15-day FOLLOW-UP | 1-MONTH FOLLOW -UP | | 2-MONTH FOLLOW -UP | | | 3-MONTH FOLLOW-UP | | 4-MONTH FOLLOW-UP | | 5-MONTH FOLLOW-UP | | 6-MONTH FOLLOW-UP | | 7-MONTH FOLLOW-UP | | CONCLUSIONS | | OBJECTIVE OF THE STUDY | |
| No adverse events. Patient satisfied | No adverse events. Patient satisfied | | Patient satisfied. Greater hair density in the treated area, stronger hair showing more color | | | NA | | Patient satisfied. Greater hair density. | | No changes or adverse events. Area controlled. | | NA | | NA | | Greater density | | Stabilize or stop hair loss and recover hair density. | |

In addition, in order to evaluate the efficacy of the cell composition of the invention in the treatment of effluviums, the composition product of Step 2 of example 3 (infiltration throughout the middle region) was administered via subepidermally to a second human subject suffering from this disease.

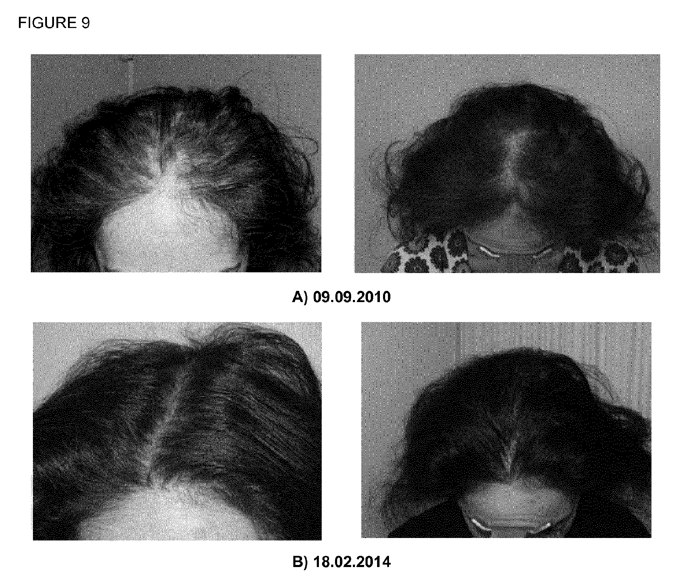

As illustrated in table XIV below and also in figure 9, after treatment, the subject did not show any adverse effects and surprisingly, after a follow-up period of 6 months the composition stabilized the hair loss and recovered hair density. For these reasons, this technique is an effective and simple alternative for the treatment of this type of alopecia for which no treatment has been demonstrated to be effective.

**Table XIV**

| **PATIENT BLP1969 (TREATMENT WITH CELLS)** | | | | | | | | | | | | | | | | | | | |
|---|---|---|---|---|---|---|---|---|---|---|---|---|---|---|---|---|---|---|---|
| INDICATION | PATIENT CODE | SEX | | AGE | TYPE OF ALOPECIA | | DISEASES | | ADVANCED MEDICAL TREATMENT DATE | | DOSE | TOTAL CELL COUNT | VOLUME OF SUSPENSION TO INJECT | | AREA TREATED (CM²) | | LOCATION OF IMPLANT | | GF INFILTRATION |
| Acquired non-scarring alopecia. Effluvium. | BLP1969 | F | | 44 | Effluvium | | No | | 01.07.2013 | | 1^{st} | 6,000,000 | 4 ml | | 49 | | Middle area | | 15 days, 1 month |

| **PATIENT BLP1969 (TREATMENT WITH CELLS)** | | | | | | | | | | | | | | | | | | | |
|---|---|---|---|---|---|---|---|---|---|---|---|---|---|---|---|---|---|---|---|
| 15-day FOLLOW-UP | 1-MONTH FOLLOW-UP | | 2-MONTH FOLLOW-UP | | | 3-MONTH FOLLOW-UP | | 4-MONTH FOLLOW-UP | | 5-MONTH FOLLOW-UP | | 6-MONTH FOLLOW-UP | | 7-MONTH FOLLOW-UP | | CONCLUSIONS | | OBJECTIVE OF THE STUDY | |
| Patient satisfied. Hair loss has stopped. | Greater hair density is observed in the frontal-parietal area | | Patient satisfied. Greater density and stronger hair. | | | No changes | | NA | | NA | | Patient satisfied and wants to repeat treatment. She is advised to wait. | | NA | | Greater hair density. Patient requests new treatment. | | Stabilize or stop hair loss and recover hair density. | |

## Claims

1. A method of obtaining a cell suspension comprising hair progenitor cells (HPCs) of a human subject which comprises the following steps:
a. Obtaining a tissue sample comprising at least one complete hair follicle from the subject, preferably from the occipital region of the head of the subject;
b. Enzymatically or mechanically digest the cells from the tissue obtained from the sample of step a);
c. If needed, inactivate the enzyme of step b); and
d. Removing the tissue fragments from the cell suspension obtained from steps b) or c), preferably by filtration.

2. A method of obtaining a cell suspension comprising hair progenitor cells (HPCs) of a human subject which comprises the following steps:
a. Obtaining a tissue sample comprising at least one complete hair follicle from the subject, preferably from the occipital region of the head of the subject;
b. Introducing the tissue obtained from step a) in a tube containing collagenase;
c. Incubating the tissue with collagenase for a period of time between 15 and 60 minutes while shaking at a temperature of approximately 37 °C;
d. Inactivating the enzyme of step c); and
e. Removing the tissue fragments from the cell suspension obtained from step d), preferably by filtration.

3. A method of obtaining a cell suspension comprising hair progenitor cells (HPCs) of a human subject which comprises the following steps:
a. Obtaining a tissue sample comprising at least one complete hair follicle from the subject, preferably from the occipital region of the head of the subject;
b. Introducing the tissue in a tube containing 0.075 % w/v of collagenase;
c. Incubating the tissue with the collagenase for a period of time between 15 and 60 minutes while shaking, preferably for a period of approximately 30 minutes, at a temperature of approximately 37 °C;
d. Inactivating the enzyme of step c), preferably with human serum;
e. Removing the tissue fragments from the product of step d);
f. Centrifuging the product of step e) and removing the supernatant.

4. A method of obtaining a composition comprising a cell suspension comprising hair progenitor cells (HPCs) from a human subject and plasma derived growth factors, which comprises the following steps:
a. Obtaining a tissue sample comprising at least one complete hair follicle from the subject, preferably from the occipital region of the head of the subject;
b. Introducing the tissue obtained from step a) in a tube containing an enzyme suitable for the enzymatic digestion of the cells;
c. Incubating the tissue with the enzyme;
d. Inactivating the enzyme of step c);
e. Removing the tissue fragments from the product of step d);
f. Re-suspending the cell composition of step e) in a suspension of plasma derived growth factors, preferably obtained from the subject; and
g. Filtrating the cell suspension obtained from step f).

5. A method of obtaining a composition comprising a cell suspension comprising hair progenitor cells (HPCs) from a human subject and plasma derived growth factors, which comprises the following steps:
a. Obtaining a tissue sample comprising at least one complete hair follicle from the subject, preferably from the occipital region of the head of the subject;
b. Introducing the tissue in a tube containing 0.075 % w/v collagenase;
c. Incubating the tissue with the collagenase for a period of time between 15 and 60 minutes while shaking at a temperature of approximately 37 °C, preferably for a period of approximately 30 minutes;
d. Inactivating the enzyme of step c), preferably with human serum;
e. Removing the tissue fragments from the product of step d);
f. Centrifuging the product of step e) and remove the supernatant;
g. Re-suspending the cell composition of step f) in a suspension of plasma derived growth factors, preferably obtained from the subject; and
h. Filtrating the cell suspension obtained of step g).

6. A method of obtaining a composition comprising a cell suspension comprising hair progenitor cells (HPCs) from a human subject and plasma derived growth factors, which comprises the following steps:
a. Obtaining a tissue sample comprising at least one complete hair follicle from the subject, preferably from the occipital region of the head of the subject;
b. Incubating the tissue in a suspension of plasma derived growth factors, preferably obtained from the subject; for a period of time between 15 minutes and 1 hour at a temperature of approximately 37 °C;
c. Removing the tissue fragments from the product of step b); preferably by filtration.

7. A method of obtaining a composition comprising a cell suspension comprising hair progenitor cells (HPCs) from a human subject and plasma derived growth factors, which comprises the following steps:
a. Obtaining a tissue sample comprising at least one complete hair follicle from the subject, preferably from the occipital region of the head of the subject;
b. Optionally, washing the tissue obtained from step a);
c. Incubating the tissue in a suspension of plasma derived growth factors, preferably obtained from the subject; for a period of time between 15 minutes and 1 hour at a temperature of 37 °C;
d. Homogenizing the cell composition of step c) by means of a mechanical or manual homogenizer such as a Potter homogenizer; and
e. Removing the tissue fragments from the product of step d); preferably by filtration.

8. A method of obtaining a composition comprising a cell suspension comprising hair progenitor cells (HPCs) from a human subject and plasma derived growth factors, which comprises the following steps:
a. Obtaining a tissue sample comprising at least one complete hair follicle from the subject, preferably from the occipital region of the head of the subject;
b. Optionally, washing the tissue obtained from step a), preferably the washing step is done more than one time;
c. Incubating the tissue in a suspension of plasma derived growth factors, preferably obtained from the subject; for a period of time between 15 minutes and 1 hour at a temperature of approximately 37 °C;
d. Homogenizing the cell composition of step c) by means of a mechanical or manual homogenizer such as a Potter homogenizer; and
e. Removing the tissue fragments from the product of step d); preferably by filtration.

9. A method of obtaining a composition comprising a cell suspension comprising hair progenitor cells (HPCs) from a human subject and plasma derived growth factors, which comprises the following steps
a. Obtaining a tissue sample comprising at least one complete hair follicle from the subject, preferably from the occipital region of the head of the subject;
b. Optionally, washing the tissue obtained from step a), preferably this washing step is done more than one time;
c. Incubating the tissue in a suspension of plasma derived growth factors, preferably obtained from the subject; for a period of time of approximately 30 minutes at a temperature of 37 °C;
d. Homogenizing the cell composition of step c) by means of a mechanical or manual homogenizer such as a Potter homogenizer; and
e. Removing the tissue fragments from the product of step d); preferably by filtration.

10. A cell suspension comprising hair progenitor cells (HPCs) of a human subject obtained or obtainable by means of the method of any of claims 1 to 3.

11. A composition comprising hair progenitor cells (HPCs) of a human subject obtained or obtainable by means of the method of any of claims 4 or 5.

12. A composition comprising hair progenitor cells (HPCs) of a human subject obtained or obtainable by means of the method of any of claims 6 to 9.

13. A cell suspension or a composition as defined in any of claims 10 to 12 for use in the treatment of hair loss or in the treatment of an inflammatory skin disorder.

14. A cell suspension or a composition as defined in any of claims 10 to 12 for use in the treatment of scarring or non-scarring alopecia.

15. A cell suspension or a composition as defined in any of claims 10 to 12 for use in the treatment of a scarring or non-scarring alopecia selected from the list consisting of hair implant scarring alopecia, scarring alopecia acquired by radiation, frontal fibrosing scarring alopecia, autoimmune non-scarring (Areata) alopecia, non-scarring follicular miniaturization alopecia and Effluviums.
